# EUROPEAN PATENT APPLICATION

(11) **EP 4 643 826 A1**
(43) Date of publication of application: **05.11.2025**
(21) Application number: 23910396.3
(22) Date of filing: 22.12.2023
(51) Int. Cl.: A61F 2/915, A61F 2/07, A61F 2/856, A61F 2/82

(54) **GROOVED STENT**

(30) Priority: 29.12.2022 CN 202211710240
(71) Applicant: Lifetech Scientific (Shenzhen) Co., Ltd., Shenzhen, Guangdong 518063 (CN)
(72) Inventor: CHEN, Erchong, Shenzhen, Guangdong 518063 (CN)
(74) Representative: Michalski Hüttermann & Partner Patentanwälte mbB
(86) International application number: PCT/CN2023/141010
(87) International publication number: WO 2024/140470

(57) **Abstract**

A grooved stent (100) is provided. Support units (33) are arranged on two sides of a mesh region (30), and the support units (33) can move relative to the mesh region (30) to achieve a certain force buffering effect under pressure. Meanwhile, a gap is reserved between each support unit (33) and the mesh region (30), which provides space for deformation under pressure, to achieve a better buffering effect and to better maintain an overall shape of a bottom support sheet (3). A grooved stent (100) is further provided. The radial size of an end close to an axial edge of a groove (5) of a bottom support sheet (3) is less than the maximum radial size of the bottom support sheet (3), which is conducive to formation of a structure for directing toward a branch port.

## Description

### Technical Field

The present disclosure relates to the technical field of medical devices, and in particular, to a rabbet or grooved stent.

### Background Art

Aortic aneurysm and aortic dissection are diseases that currently pose a serious threat to human life. Without timely and effective treatment, aortic aneurysms and dissections can progressively enlarge and ultimately rupture, leading to severe complications and death. With the increasing number of patients with hypertension, hyperlipidemia, and hyperglycemia, the incidence of aortic aneurysm and aortic dissection is also increasing significantly.

Conventional open surgery for treating aortic aneurysm and aortic dissection has the disadvantages of a large wound, high mortality, long operation duration, high incidence of postoperative complications, and high operation difficulty. Intracavitary therapy has the advantages of a smaller wound, few postoperative complications, short operation duration, and low operation difficulty, and has gradually become the main treatment method of aortic aneurysm and aortic dissection. By implanting a lumen stent in the aorta, vascular lesions are isolated outside the lumen stent and blood flow is directed to flow through the lumen stent, thereby achieving the purpose of protecting the blood vessel.

When an aneurysm or arterial dissection is located near a branch blood vessel of the aorta, the implanted lumen stent may obstruct an opening of the branch blood vessel, thereby impeding blood flow in the branch blood vessel. One of the current methods for solving this problem is to form a groove in the stent, with a window formed in the groove to allow blood flow. The window corresponds to the branch blood vessel, ensuring that blood in the aorta can flow through the window and flow in the branch blood vessel. Typically, the bottom of the groove is covered with a membrane to occlude the channel of a main stent. After being implanted in a blood vessel, the main stent is compressed by the blood vessel in a radial direction, and the bottom of the groove is prone to deformation. This deformation may obstruct a branch port, compress groove space, and hinder a passage of a guide wire or bridging stent into a branch.

### Summary of the Disclosure

In view of the deficiencies in the prior art, the present disclosure provides a rabbet or grooved stent, which is of a hollow tubular structure with ports at two ends and includes a main stent and a bottom support sheet. A groove is formed in a surface of the main stent, and the bottom support sheet is arranged at the bottom of the groove and close to an axial edge of the groove. The bottom support sheet includes a mesh region. A radial side of the mesh region is connected to at least one deformable support unit, the support unit includes at least one movable end portion, a gap is reserved between the movable end portion and the mesh region, and the movable end portion is capable of moving relative to the mesh region at least in a radial direction.

In an embodiment, the support unit includes a fixed end portion, and is connected to the mesh region through the fixed end portion.

In an embodiment, the support unit includes a connecting rod, one end of the connecting rod is connected to the mesh region to form the fixed end portion, the other end of the connecting rod extends away from the mesh region and toward a radial edge of the groove, to form the gap with the mesh region.

In an embodiment, the support unit further includes a buffer rod connected to the connecting rod, and the buffer rod is capable of moving relative to the connecting rod.

In an embodiment, the support unit includes two connecting rods and at least one buffer rod, wherein the ends connected to the mesh region of the two connecting rods are intersected to form the fixed end portion, the other ends of the two connecting rods extend obliquely from the fixed end portion in directions away from each other and are respectively connected to two ends of the buffer rod.

In an embodiment, the support unit includes at least two connecting rods and a plurality of buffer rods, the plurality of buffer rods are connected end to end to form a polygonal rod. The ends connected to the mesh region of two connecting rods are intersected to form the fixed end portion, the other ends of the two connecting rods extend obliquely from the fixed end portion in directions away from each other and are respectively connected to head and tail ends of the polygonal rod to form a polygonal grid.

In an embodiment, the included angle between one connecting rod and the buffer rod is greater than or equal to the included angle between the other connecting rod and the buffer rod.

In an embodiment, the lengths of the two connecting rods are equal or the length of a connecting rod is greater than the length of the other connecting rod.

In an embodiment, the minimum distance between the end away from the mesh region of one connecting rod and the mesh region is greater than the minimum distance between the end away from the mesh region of the other connecting rod and the mesh region.

In an embodiment, the mesh region includes a first row of grids and a second row of grids that are adjacent to each other in an axial direction, the first row of grids are closer to the axial edge of the groove compared with the second row of grids, and the support unit is connected to a radial side of the second row of grids.

In an embodiment, the first row of grids include a plurality of first grids arranged in the radial direction; the second row of grids include a plurality of second grids arranged in the radial direction; and one first grid corresponds to one second grid in the axial direction, and the quantity of first grids is less than or equal to the quantity of second grids.

In an embodiment, the first row of grids include side grids on two radial sides and a middle grid between the two side grids, and thr distance between the middle grid and the axial edge of the groove is less than the distance between each side grid and the axial edge of the groove.

The present disclosure has the following beneficial effects: compared with the prior art, according to the grooved stent provided in the present disclosure, the support units are arranged on two sides of the mesh region, and the support units can move relative to the mesh region to achieve a certain force buffering effect under pressure. Meanwhile, the gap is reserved between each support unit and the mesh region, which provides space for deformation under pressure, to achieve a better buffering effect and to better maintain an overall shape of the bottom support sheet.

The present disclosure provides a grooved stent, which is of a hollow tubular structure with ports at two ends and includes a main stent and a bottom support sheet. A groove is formed in a surface of the main stent, and the bottom support sheet is arranged at the bottom of the groove and close to an axial edge of the groove. The radial size of an end close to the axial edge of the groove of the bottom support sheet is less than the maximum radial size of the bottom support sheet.

In an embodiment, the bottom support sheet includes a mesh region, which includes a first row of grids and a second row of grids that are adjacent to each other in an axial direction; and the first row of grids are closer to the axial edge of the groove compared with the second row of grids.

In an embodiment, the radial size of the first row of grids is less than or equal to the radial size of the second row of grids.

In an embodiment, the radial size of the first row of grids is less than the radial size of the axial edge of the groove.

In an embodiment, the bottom support sheet includes a first mesh region and two second mesh regions respectively connected to two sides of the first mesh region in a radial direction; the first mesh region and the second mesh regions include the first row of grids and the second row of grids that are adjacent to each other in the axial direction; the first row of grids and the second row of grids are hooked in the two second mesh regions, and the first row of grids and the second row of grids are connected in the first mesh region to form cross units.

In an embodiment, the main stent includes a proximal section, a distal section, and a middle section between the proximal section and the distal section. The groove is formed in the middle section. A branch stent is arranged in an inner cavity of the proximal section and/or the distal section and close to the groove, and includes a branch port. The bottom support sheet is arranged in the groove, and at least an axial end of the bottom support sheet is arranged close to the branch port.

In an embodiment, the branch stent includes a stent with two branch ports; and the bottom support sheet close to the stent with two branch ports is a first bottom support sheet. The stent with two branch ports includes a first branch stent and a second branch stent that are arranged side by side in the radial direction, a first branch port is formed at an end close to the groove of the first branch stent, a second branch port is formed at an end close to the groove of the second branch stent, wherein edges on sides that are away from an inner surface of the main stent of the first branch port and the second branch port form protruding gaps bulging toward the inner surface of the main stent, and the first mesh region of the first bottom support sheet is arranged opposite to the protruding gaps in the axial direction.

In an embodiment, the bottom support sheet includes the mesh region; a radial side of the mesh region is connected to at least one deformable support unit, the support unit includes at least one movable end portion, a gap is reserved between the movable end portion and the mesh region, and the movable end portion is capable of moving relative to the mesh region in the radial direction.

In an embodiment, the support unit includes a fixed end portion, a connecting rod, and a buffer rod connected to the connecting rod, and is connected to the mesh region through the fixed end portion, one end of the connecting rod is connected to the mesh region to form the fixed end potion, the other end of the connecting rod extends away from the mesh region and toward a radial edge of the groove, to form the gap with the mesh region, and the buffer rod is capable of moving relative to the connecting rod.

In an embodiment, the support unit includes a fixed end portion, at least two connecting rods, and at least one buffer rod, and is connected to the mesh region through the fixed end portion, wherein the ends connected to the mesh region of two connecting rods are intersected to form the fixed end portion, the other ends of the two connecting rods extend obliquely from the fixed end portion in directions away from each other and are respectively connected to two ends of the buffer rod.

The present disclosure has the following beneficial effects: the overall bottom support sheet is a structure for directing toward the branch port, which can better guide a guide wire and a bridging stent into the branch port. The structure is similar to a structure of a transition section between the groove and the branch port, which ensures high adaptability of the bottom support sheet.

The present disclosure provides a grooved stent, which includes a bottom support sheet. The bottom support sheet includes a first mesh region and two second mesh regions respectively connected to two sides of the first mesh region in a radial direction; the first mesh region and the second mesh regions include a first row of grids and a second row of grids that are adjacent to each other in an axial direction; the first row of grids and the second row of grids are hooked in the two mesh regions, and the first row of grids and the second row of grids are connected in the first mesh region to form cross units.

In an embodiment, the first row of grids include a plurality of first grids arranged in the radial direction; the second row of grids include a plurality of second grids arranged in the radial direction; and one first grid corresponds to one second grid in the axial direction, and the first grids in the second mesh regions are all hooked to the corresponding second grids to form hook unit.

In an embodiment, the first mesh region includes a first directional wave rod and a second directional wave rod that are overlapped with each other to form the cross unit.

In an embodiment, the grooved stent further includes a main stent, which includes a proximal section, a distal section, and a middle section between the proximal section and the distal section. A groove is formed in the middle section. A branch stent is arranged in an inner cavity of the proximal section and/or the distal section and close to the groove, and includes a branch port. The bottom support sheet is arranged in the groove, and at least an axial end of the bottom support sheet is arranged close to the branch port.

In an embodiment, the axial end close to the branch port of the bottom support sheet is spaced apart from or abuts against an edge of the branch port.

In an embodiment, the branch stent includes a stent with two branch ports; and the bottom support sheet close to the stent with two branch ports is a first bottom support sheet.

In an embodiment, the stent with two branch ports includes a first branch stent and a second branch stent that are arranged side by side in the radial direction, a first branch port is formed at an end close to the groove of the first branch stent, a second branch port is formed at an end close to the groove of the second branch stent, and edges on sides that are away from an inner surface of the main stent of the first branch port and the second branch port form protruding gaps bulging toward the inner surface of the main stent.

In an embodiment, the first mesh region of the first bottom support sheet is arranged opposite to the protruding gaps in the axial direction.

In an embodiment, the second mesh region of the first bottom support sheet is opposite to the first branch stent and the second branch stent in the axial direction.

In an embodiment, the grooved stent further includes an imaging unit, which is located in an axial region where the first bottom support sheet is located, and is arranged opposite to the protruding gaps in the axial direction.

In an embodiment, the bottom support sheet sinks relative to an opening of the groove toward an inner cavity of the main stent to form an arc-shaped structure.

In an embodiment, at least part of the first mesh region of the bottom support sheet forms a protruding portion on a concave surface of the arc-shaped structure.

In an embodiment, a main membrane is arranged on a surface of the main stent; a bottom membrane is arranged at the bottom of the groove, an axial edge of the bottom membrane is connected to the branch port, and a radial edge of the bottom membrane is connected to the main membrane.

In an embodiment, the bottom membrane covers an outer surface of the bottom support sheet.

In an embodiment, the branch stent includes a stent with a single branch port; and the bottom support sheet close to the stent with a single branch port is a second bottom support sheet.

In an embodiment, the first mesh region of the second bottom support sheet is arranged opposite to a branch port of the stent with a single branch port.

In an embodiment, the grooved stent includes two bottom support sheets arranged at intervals and a middle support sheet between the two bottom support sheets in the axial direction. In the middle support sheet, at least two waveform units are overlapped with each other to form at least one row of grid support sections extending in a circumferential direction.

In an embodiment, the grid support sections are provided with a plurality of third grids in the radial direction, and the wave width of the third grid in a region between two radial sides of the grid support sections is less than or equal to the wave widths of the third grids on two radial sides.

In an embodiment, each waveform unit of the grid support sections includes a high wave and a low wave, the axial length of the high wave is greater than or equal to the axial length of the low wave, and the high wave is arranged opposite to or staggered with an edge wave angle of the adjacent support sheet.

In an embodiment, the middle support sheet protrudes relative to the opening of the groove away from the inner cavity of the main stent to form an arc-shaped structure.

In an embodiment, the bottom support sheet is prepared by integrated weaving of shape memory metal wires, or prepared by integrated cutting of a shape memory metal material.

The present disclosure has the following beneficial effects: compared with the prior art, the grooved stent provided in the present disclosure includes the bottom support sheet, which includes the first mesh region and the two second mesh regions connected to two sides of the first mesh region. Arrangement of the bottom support sheet is conducive to maintenance of the shape of the bottom of the groove, and can reduce the risk of obstructing the branch port by the bottom of the groove under radial compression. In addition, because the structure of the first mesh region has better deformation capability compared with the structure of the second mesh region, when the bottom support sheet is subjected to a radial force, the second mesh region can well resist the radial force, to maintain the overall shape of the bottom of the groove and prevent the bottom of the groove from being deformed significantly. Furthermore, the first mesh region has high adaptability to the branch port, which prevents the bottom of the groove from obstructing the branch port under compression and prevents a bridging stent connected to the branch stent from being deformed under compression.

### Brief Description of the Drawings

FIG. 1 is a schematic diagram of an overall structure of a grooved stent of the present disclosure;
FIG. 2 is a schematic diagram of an unfolded bottom support sheet of the present disclosure;
FIG. 3 is a schematic structural diagram of an imaging unit on a bottom support sheet of the present disclosure;
FIG. 4 is a partial enlarged view of section A in FIG. 2 of the present disclosure;
FIG. 5 is a partial enlarged view of section B in FIG. 2 of the present disclosure;
FIG. 6 is a partial enlarged view of section C in FIG. 2 of the present disclosure;
FIG. 7 is a schematic structural diagram of a distal bottom support sheet in Embodiment IV of the present disclosure;
FIG. 8 is a schematic structural diagram of a bottom support sheet in Embodiment II of the present disclosure;
FIG. 9 is a schematic structural diagram of a bottom support sheet in Embodiment **III** of the present disclosure;
FIG. 10 is a schematic diagram of a projection of a bottom support sheet onto a radial plane of the present disclosure;
FIG. 11 is a schematic structural diagram of a protruding portion formed by a bottom support sheet on a concave surface of the present disclosure;
FIG. 12 is a schematic structural diagram of a W-like shape formed by a bottom support sheet of the present disclosure;
FIG. 13 is a schematic diagram showing adaptation of a bottom support sheet and a stent with two branch ports of the present disclosure;
FIG. 14 is a schematic structural diagram of a bottom support sheet and a stent with two branch ports of the present disclosure;
FIG. 15 is a schematic structural diagram of a bottom support sheet and a stent with a single branch port of the present disclosure;
FIG. 16 is a schematic structural diagram of a middle support sheet of the present disclosure;
FIG. 17 is a schematic diagram of a projection of a middle support sheet onto a radial plane of the present disclosure;
FIG. 18 is a schematic structural diagram of a middle section of a grooved stent of the present disclosure;
FIG. 19 is a schematic diagram of staggered high waves of a middle support sheet of the present disclosure;
FIG. 20 is a schematic diagram of opposite high waves of a middle support sheet of the present disclosure;
FIG. 21 is a schematic diagram of some opposite high waves and some staggered high waves of a middle support sheet of the present disclosure;
FIG. 22 is a schematic diagram of opposite directions of arc-shaped structures of a middle support sheet and a bottom support sheet of the present disclosure; and
FIG. 23 is a schematic diagram of a same direction of arc-shaped structures of a middle support sheet and a bottom support sheet of the present disclosure.

### Detailed Description of the Disclosure

To better understand the concept of the present disclosure, implementations of the present disclosure are specifically described below with reference to the drawings. The following specific embodiments are merely some embodiments of the present disclosure, and are not intended to limit the present disclosure.

For ease of description, spatial relative relationship terms may be used herein to describe a relationship of one element or feature relative to another element or feature as shown in a figure. These relative relationship terms include, for example, "interior", "exterior", "inside", "outside", "under", "below", "on", and "above". Such spatial relative relationship terms are intended to encompass different orientations of an apparatus in use or operation in addition to an orientation depicted in the figure. For example, if the apparatus in the figure is turned over, an element described as "under" or "below" another element or feature would then be oriented "on" or "above" the another element or feature. Thus, the exemplar term "below" can encompass both an orientation of above and below. The apparatus may be oriented in another way (rotated 90 degrees or at another orientation) and is accordingly interpreted by using a spatial relative relationship descriptor herein.

To more clearly describe a structure of the present application, the terms "proximal" and "distal" are defined herein as commonly used terms in the field of interventional medicine. Specifically, a "distal end" refers to an end where blood flows out, and a "proximal end" refers to an end where blood flows in. For example, after a stent is implanted, blood flows from a proximal end of the stent to a distal end of the stent. An "axial direction" refers to a length direction of the stent, and a "radial direction" refers to a direction perpendicular to the "axial direction". A radial direction of a groove or a bottom support sheet refers to a width direction of the groove or the bottom support sheet.

In the present disclosure, a "wave ring" (also called a waveform loop) is a closed ring structure, and a "waveform unit" is an arc-shaped structure. The "wave ring" and the "waveform unit" are prepared by weaving or cutting a metal elastic material or a polymer material. The metal elastic material includes materials known in the field of implantable medical instruments or a combination of various biocompatible materials, such as an alloy of two or more of cobalt, chromium, nickel, titanium, magnesium, and iron, 316L stainless steel, or a tantalum-nickel-tantalum alloy, or other biocompatible metal elastic materials. The polymer materials include biocompatible materials such as polylactic acid. The "wave ring" and the "waveform unit" both have radial expansion capabilities, can contract in a radial direction under an external force, and can self-expand or can be mechanically expanded (for example, by balloon expansion) to restore to an original shape and maintain the original shape after the external force is withdrawn. In this way, after being implanted into a lumen, a stent is adhered to an inner wall of the lumen by virtue of a radial support force of the wave ring or the waveform unit. A shape of a wave in the "wave ring" or the "waveform unit" is not limited, and the wave may be a Z-shaped wave, an M-shaped wave, a V-shaped wave, a sine wave, or the like. The "wave ring" and the "waveform unit" both include a plurality of crests (also called proximal vertices), a plurality of troughs (also called distal vertices), and wave rods for connecting the crests to the adjacent troughs. One vertex (the proximal vertex or the distal vertex) and two wave rods connected to the vertex form one wave.

In the present disclosure, a "membrane" can isolate liquid to a certain extent, which may be made from a biocompatible polymer material such as polytetrafluoroethylene (PTFE) or polyethylene terephthalate (PET).

### Embodiment I

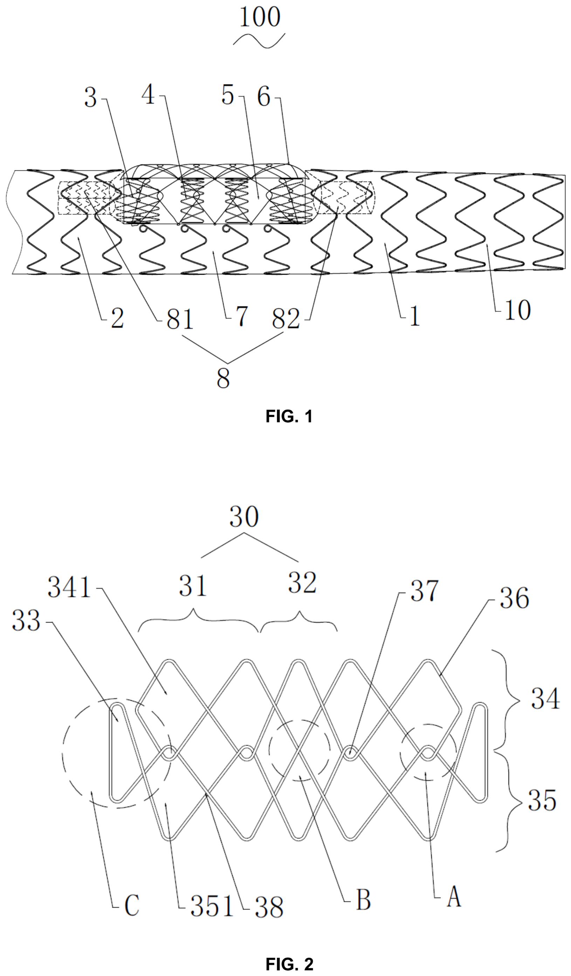

Referring to FIG. 1, in this embodiment, an entire grooved stent 100 is of a hollow tubular structure with ports at two ends, and includes a main stent 10.

A surface of the main stent 10 is covered with a main membrane 11. The main membrane 11 may be arranged on an inner surface and/or an outer surface of the main stent 10, or the main membrane 11 may be arranged on a part of the inner surface and a part of the outer surface of the main stent 10.

Referring to FIG. 1, the main stent 10 may be divided into a proximal section 2, a distal section 1, and a middle section 7 between the proximal section 2 and the distal section 1. The proximal section 2 includes a tubular proximal stent and a proximal main membrane 11, and the proximal main membrane 11 may be covered on an inner side or an outer side of the proximal stent by suturing, bonding, hot melting, or another method. The proximal stent includes a plurality of main wave rings arranged at intervals in an axial direction. The distal section 1 includes a tubular distal stent and a distal main membrane 11, and the distal main membrane 11 may be covered on an inner side and/or an outer side of the distal stent by suturing, bonding, hot melting, or another method. The distal stent includes a plurality of main wave rings arranged at intervals in the axial direction.

The middle section 7 includes a middle unit and at least one mesh cover 6. The middle unit includes a middle main membrane 11 and a middle stent (which may be omitted in other embodiments). An inner cavity enclosed by the middle main membrane 11 communicates with an inner cavity enclosed by the proximal main membrane 11 and an inner cavity enclosed by the distal main membrane 11. A groove 5 is formed in a surface of the middle unit. Two circumferential sides of the mesh cover 6 are respectively fixedly connected to the middle main membrane 11 by suturing, bonding, hot melting, or another method, and a gap (also called a clearance or cavity) is reserved between at least part of the mesh cover 6 and an outer surface of the middle unit and communicates with an inner cavity of a branch stent 8. A plurality of branch stents 8 are respectively arranged close to the proximal end of the groove 5 and close to the distal end of the groove 5.

In an embodiment, the mesh cover 6 is of an arc-shaped structure in a circumferential direction, which is a mesh support structure prepared by integrated weaving of weaving wires. The central angle corresponding to a projection of the mesh cover 6 onto a radial plane is less than or equal to 120 degrees, so that the mesh cover 6 has a good radial support force, and sufficient space is reserved in the middle unit for blood flow.

In this embodiment, an edge formed on the main membrane 11 of the groove 5 is rectangular, that is, when the main membrane 11 is unfolded along a generatrix that does not pass through the groove 5, the groove 5 is rectangular. The groove 5 has a first edge, a second edge, a third edge, and a fourth edge, and is enclosed by the four edges. The first edge and the second edge are opposite to each other and in the same direction as a length extension direction of the grooved stent 100, and the third edge and the fourth edge are opposite to each other in the axial direction and are closer to an end portion of the grooved stent 100 compared with the first edge and the second edge. It may be understood that in other embodiments, the groove 5 may be in other shapes, as long as the first edge and the second edge extend along the length extension direction of the grooved stent 100. For example, a certain angle is formed between the third edge or the fourth edge and the length extension direction of the grooved stent 100 (for example, the groove 5 is trapezoidal), or the first edge and second edge are arcs (for example, the groove 5 is approximately oval). The specific shape of the groove 5 is not limited in the present disclosure. It may also be understood that the groove 5 may be close to a proximal end or a distal end of the grooved stent 100.

Referring to FIG. 2, the grooved stent 100 includes bottom support sheets 3 that are close to the branch stent 8 and that are respectively arranged at the proximal end and the distal end of the groove 5. That is, the bottom support sheets 3 are arranged in the groove 5 and close to the branch stent 8. The bottom support sheets 3 on two sides have a same structure and are prepared by mutual overlapping and/or mutual hooking of weaving wires. After being woven, the weaving wires are connected end to end to form a mesh woven structure. Each bottom support sheet 3 includes a mesh region 30 formed by mutual overlapping and/or mutual hooking of a plurality of waveform units 36. A radial side of the mesh region 30 is connected to at least one deformable support unit 33. The support unit 33 is formed by weaving two sides of the woven structure of the bottom support sheet 3 in a radial direction of the mesh region 30. The support unit 33 is connected to the mesh region 30 through a fixed end portion 334 and includes at least one movable end portion 335. A movable gap 333 is reserved between the movable end portion 335 and the mesh region 30. The movable end portion 335 can move relative to the mesh region 30 in at least the radial direction. The support unit 33 is close to the first edge and the second edge of the groove 5 in the radial direction, namely, two radial sides. Therefore, when the groove 5 of the main stent 10 is subjected to radial compression, the support unit 33 is first subjected to the force, and is deformed and move. At this point, because the movable end portion 335 is not in direct contact or connection with the mesh region 30, the support unit 33 subjected to the force does not immediately transfer the force to the mesh region 30, so that the overall shape of the mesh region 30 can be well maintained.

In an embodiment, the support unit 33 includes a connecting rod 331 (not shown in the figure). One end of the connecting rod 331 is connected to the mesh region 30 to form the fixed end portion 334, and the other end of the connecting rod 331 extends away from the mesh region 30 and toward a radial edge of the groove 5, to form the gap 333 with the mesh region 30. The connecting rod 331 is connected to the mesh region 30 and then extends away from the mesh region 30, to form the gap 333 with the mesh region 30 in a case that the other end is far enough away from the mesh region. The gap 333 can ensure a distance for a displacement activity of the connecting rod 331 under a radial force, form buffer space to achieve a buffering effect, and ensure that the force is not directly transferred to the mesh region 30, so that the overall shape of the mesh region 30 can be well maintained.

In another embodiment, the end that is away from the mesh region 30 of the connecting rod 331 is connected to one buffer rod 332 (not shown in the figure). Preferably, the buffer rod 332 is arranged parallel to or at an angle to the radial edge of the groove 5. The buffer rod 332 is connected to the membrane at this position. Further, the buffer rod 332 can be first deformed under the radial force to buffer the radial force, and then transfer the force that cannot be buffered to the connecting rod 331. The connecting rod 331 moves to further buffer the radial force, which ensures that the force is not directly transferred to the mesh region 30.

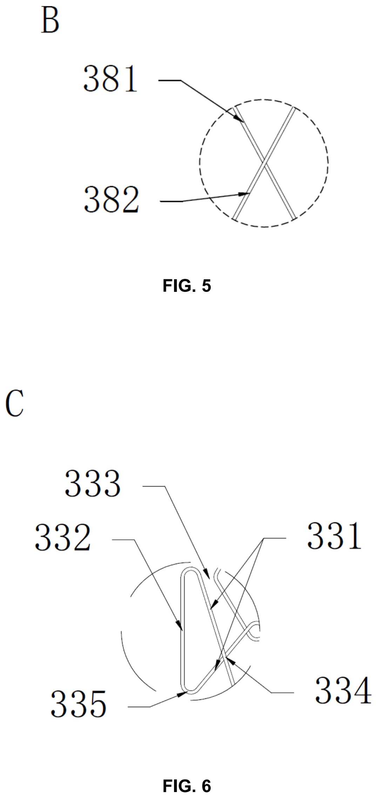

Referring to FIG. 2 and FIG. 6, in another embodiment, the support unit 33 includes at least two connecting rods 331 and at least one buffer rod 332. One end connected to the mesh region 30 of two connecting rods 331 are intersected to form the fixed end potion 334, and the other ends of the two connecting rods 331 extend obliquely from the fixed end portion 334 in directions away from each other and are respectively connected to two ends of the buffer rod 332. In this embodiment, the two connecting rods 331 are first intersected and then are fixedly connected to the mesh region 30. Through such an arrangement, the two connecting rods 331 can slide relative to each other to a certain extent at the intersection, to ensure that the bottom support sheet 3 easily slides and is deformed at the intersection under the radial force, thereby preventing the radial force from being directly transferred to the mesh region 30 and maintaining the overall shape. The movable end portions 335 are formed at two ends, connected to the two connecting rods 331, of the buffer rod 332. The movable end portion 335 first moves under the radial force to form a first-stage buffer structure, which prevents excessive deformation of the overall shape of the mesh region 30 from affecting internal space of a main channel.

Further referring to FIG. 2, in the mesh regions 30, a first row of grids 34 and a second grow of grids 35 that are adjacent to each other in the axial direction are formed by mutual overlapping and hooking of the waveform units 36. The first row of grids 34 are closer to an axial edge of the groove 5 compared with the second row of grids 35. The support unit 33 is connected to a radial side of the second row of grids 35. When the bottom support sheet 3 is installed at the bottom of the groove 5, the first row of grids 34 are closer to a branch port of the branch stent 8 compared with the second row of grids 35.

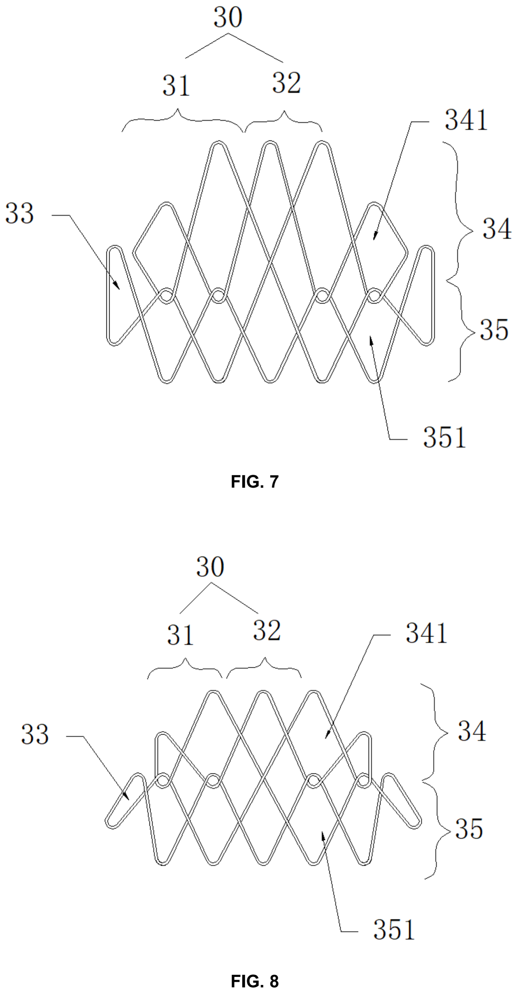

Referring to FIG. 2 and FIG. 7, it may be understood that when one buffer rod 332 is arranged, a triangular grid is enclosed by the two connecting rods 331 and the buffer rod 332 of the support unit 33. The triangular grids are located at joints between the bottom support sheet 3 and two radial edges of the groove 5, that is, located at the first edge and the second edge of the groove 5. Specifically, the triangular grid is connected to the second row of grids 35, the entire triangular grid is located in the middle area of the first row of grids 34 and the second row of grids 35, and forms trapezoid-like structure with the two rows of grids. Through the structures, the entire bottom support sheet 3 is a structure for directing toward the branch port. This structure is similar to a structure of a transition section between the groove 5 and the branch port, so that the bottom support sheet 3 has high adaptability. The weaving wires on two sides of the second row of grids 35 cross in opposite directions to form the fixed end portion 334 of the triangular grid, and extend away from each other and then extend toward each other and are connected to form the movable end portions 335 at two corners. The movable portions 335 at two corners respectively extend in the axial direction of the main stent 10. The side connecting the two corners is the buffer rod 332. Through the two triangular grids, when the bottom support sheet 3 is subjected to the radial force, the cross structure at the fixed end portion 334 first slides and moves to buffer part of the force, and the triangular grids move toward the central axis in the radial direction, to further buffer the radial force, thereby effectively maintaining the edge shape and the overall shape of the bottom support sheet 3.

### Embodiment II

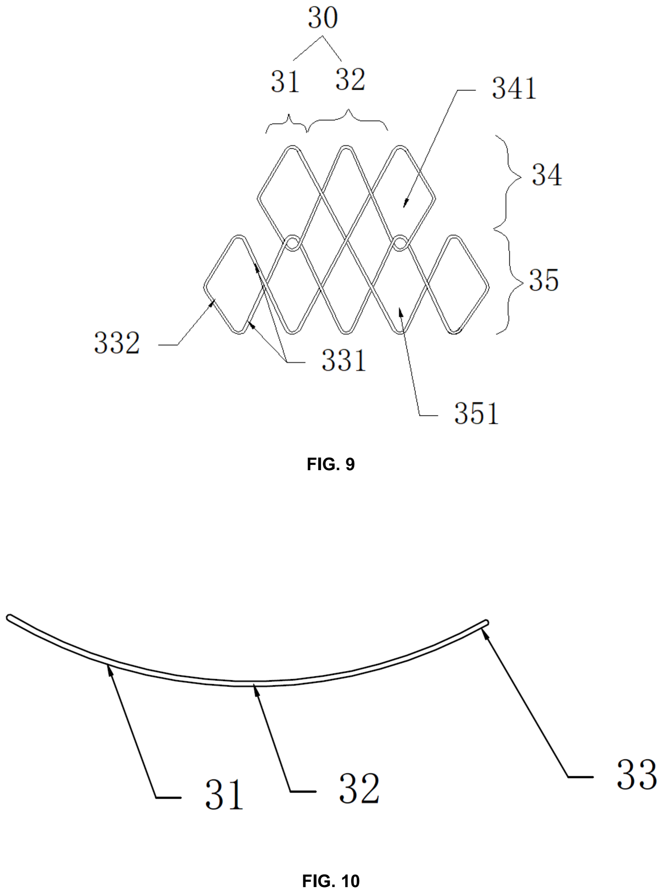

Referring to FIG. 9, the structure of a bottom support sheet 3 is basically the same as that of the bottom support sheet in Embodiment I. A support unit 33 includes a plurality of buffer rods 332, which are connected end to end to form a polygonal rod. One end connected to a mesh region 30 of two connecting rods 331 are intersected to form a fixed end portion 334, and the other ends of the two connecting rods 331 extend obliquely from the fixed end portion 334 in directions away from each other and are respectively connected to head and tail ends of the polygonal rod to form a polygonal grid. Preferably, two buffer rods 332 may be arranged, and an included angle is formed between the two connecting rods 331, to form a wave angle structure. In this way, the two buffer rods 332 and the two connecting rods 331 are connected to form a quadrilateral grid. The two connecting rods 331 connected to a second grid 351 of the quadrilateral grid can also slide relative to each other, so that the two connecting rods 331 can achieve a buffering effect under a radial force. Different from a triangular grid, the two buffer rods 332 and the formed wave angle structure define a second row of grids 35 and a first row of grids 34 to form a trapezoid-like structure. An objective of the arrangement of the trapezoid-like structure is the same as that in Embodiment I. A branch port of branch stents 8 is retracted at two ends of the groove 5. Radial lengths of a third edge and a fourth edge of the groove 5 are greater than the radial length of the branch port of the branch stent 8, thereby forming a blank section of a trapezoid-like structure. The bottom support sheet 3 is arranged as the trapezoid-like structure. Through the arrangement of this structure, the entire bottom support sheet 3 is a structure for directing toward the branch port. This structure is similar to a structure of a transition section between the groove 5 and the branch port, so that the bottom support sheet 3 has high adaptability.

### Embodiment III

Referring to FIG. 7 to FIG. 9, the structure of a bottom support sheet 3 is basically the same as that of the bottom support sheet in Embodiment I. The included angle between one connecting rod 331 and a buffer rod 332 is greater than or equal to the included angle between the other connecting rod 331 and the buffer rod 332. Referring to FIG. 9, preferably, if the included angles between the two connecting rods 331 and the buffer rod 332 are equal, when a support unit 33 is subjected to a radial force, movable end portions 335 formed by connecting the two connecting rods 331 to the buffer rod 332 move simultaneously to buffer the force, so as to achieve a uniform radial force buffering effect. If the included angles between the two connecting rods 331 and the buffer rod 332 are different, the radial force applied to the support unit 33 is usually guided in an expected direction, so that the movable end portion 335 having a smaller angle has a larger deformation amplitude. Preferably, the movable end portion 335 closer to a first row of grids 34 of a mesh region 30 of the support unit 33 has the smaller angle, so that the support unit 33 moves toward the first row of grids 34 under the radial force. In this way, the bottom support sheet 3 is deformed into a desired shape, such as a trapezoid-like shape.

Referring to FIG. 8, in another preferred solution, the movable end portion 335 closer to the second row of grids 35 of the mesh region 30 of the support unit 33 has the smaller angle, and the buffer rod 332 is inclined from the movable end portion 335 in a direction away from the second row of grids 35. Through the arrangement of this structure, a triangular grid can form a trapezoid-like structure with the entire bottom support sheet 3 without being subjected to the radial force.

In another embodiment, lengths of the two connecting rods 331 are equal or different. Referring to FIG. 9, preferably, if the lengths of the two connecting rods 331 are equal, when the support unit 33 is subjected to the radial force, the movable end portions 335 formed by connecting the two connecting rods 331 to the buffer rod 332 move simultaneously to buffer the force, so as to achieve a uniform radial force buffering force. Referring to FIG. 2 and FIG. 7, if the length of the connecting rod 331 closer to the first row of grids 34 of the mesh region 30 of the support unit 33 is greater than the length of the other connecting rod 331, the support unit 33 moves toward the first row of grids 34 under the radial force. In this way, the bottom support sheet 3 is deformed into a desired shape, such as a trapezoid-like shape.

In another embodiment, the minimum distance between an end away from the mesh region 30 of one connecting rod 331 and the mesh region 30 is greater than the minimum distance between an end away from the mesh region 30 of the other connecting rod 331 and the mesh region 30. Preferably, the minimum radial distance between an end away from the mesh region 30 of the connecting rod 331 closer to the second row of grids 35 and the mesh region 30 is greater than the minimum radial distance between an end away from the mesh region 30 of the connecting rod 331 closer to the first row of grids 34 and the mesh region 30. Through such an arrangement, when subjected to the radial force, the connecting rod 331 closer to the second row of grids 35 is prone to abut against the first row of grids 34 under the radial force, so that the first row of grids 34 is deformed in a radial direction earlier and more easily. A movement distance is reserved between a wave close to a branch port in an axial direction of the first row of grids 34 in a natural state and the branch port. Therefore, when deformed under the radial force, the bottom support sheet 3 is compressed into a sheath tube along with a grooved stent 100, so that the bottom support sheet does not excessively press against the branch port and puncture a membrane at this position.

### Embodiment IV

Referring to FIG. 8 and FIG. 9, the structure of a bottom support sheet 3 is basically the same as those of the bottom support sheets in Embodiment I to Embodiment III. A first row of grids 34 include a plurality of first grids 341 arranged in a radial direction; and a second row of grids 35 include a plurality of second grids 351 arranged in the radial direction. One first grid 341 corresponds to one second grid 351 in an axial direction. To adapt to the structure of a blank section between a branch port of a branch stent 8 and a groove 5, the quantity of first grids 341 is less than or equal to the quantity of second grids 351. If the quantity of first grids 341 is less than the quantity of second grids 351, the entire bottom support sheet 3 can better form a trapezoid-like structure.

Referring to FIG. 7, in another embodiment, if the quantity of first grids 341 is equal to the quantity of second grids 351, the axial size of a crest of at least a middle grid (the first grid 341) between two side grids in the first row of grids 34 is greater than the axial sizes of crests of two side grids (the second grids 351) in the second row of grids 35. Alternatively, it may be understood as that the first row of grids include side grids on two radial sides and a middle grid between the two side grids, and the distance between the middle grid and an axial edge of the groove is less than the distance between each side grid and the axial edge of the groove. The axial size herein refers to the size of a vertical distance between a crest and a trough of one grid. Through the arrangement of this structure, the entire bottom support sheet 3 can well form a trapezoid-like structure.

It may be understood that the distance between the middle grid in the first row of grids 34 and the axial edge of the groove 5 is not affected by the quantity of first grids 341 and the quantity of second grids 342 (not shown in the figure). Even if the quantity of first grids 341 is less than the quantity of second grids 351, the distance between the middle grid and the axial edge of the groove 5 can also be set to be less than the distance between each side grid and the axial edge of the groove 5.

Preferably, in the axial direction, each of the first grid 341 and the second grid 351 includes a proximal wave and a distal wave that are connected to each other, and the first grid 341 or second grid 351 of a polygonal grid structure is enclosed by the proximal wave and the distal wave. Preferably, the first grid 341 and the second grid 351 are quadrilateral grids. The structure of the quadrilateral grid can enhance wall adhesion and support of the bottom support sheet 3 in the groove 5. It may be understood that in other embodiments, a greater number of first grids 341 and second grids 351 indicate a smaller mesh hole size. Therefore, the quantity of grids in the same row is preferably 3-7.

Referring to FIG. 9, in an embodiment, the quantity of first grids 341 in the first row of grids 34 is 3, and the quantity of second grids 351 in the second row of grids 35 is 5-7, and specifically 5.

### Embodiment V

The structure of a bottom support sheet 3 is basically the same as those of the bottom support sheets in Embodiment I to Embodiment IV. Referring to FIG. 2 and FIG. 5 again, a mesh region 30 includes a first mesh region 32 formed by mutual overlapping of waveform units 36 and a second mesh region 31 formed by mutual hooking of the waveform units 36. At least two mesh regions 31 are arranged and are respectively connected to two sides of the first mesh region 32. A support unit 33 is connected to the second mesh region 31. The first mesh region 32 and the second mesh region 31 both can contract in a radial direction under an external force, and can self-expand or can be mechanically expanded (for example, by balloon expansion) to restore to an original shape and maintain the original shape after the external force is withdrawn.

The bottom support sheet 3 includes one first mesh region 32 and two second mesh regions 31 in the radial direction. The two second mesh regions 31 are respectively arranged on two radial sides of the first mesh region 32. In a natural unfolded state, the bottom support sheet 3 is bent from the central axis toward two side edges in the radial direction, to form an arc-shaped structure. Specifically, the arc-shaped structure sinks relative to an opening of the groove 5 toward an inner cavity of the main stent 10. The first mesh region 32 of the bottom support sheet 3 includes a plurality of first directional wave rods 381 arranged at intervals and a plurality of second directional wave rods 382 arranged at intervals. The first directional wave rods 381 extend substantially in a first direction, and the second directional wave rod 382 extend substantially in a second direction. The first directional wave rods 381 are overlapped (or interweaved) with the second directional wave rods 382 to form a plurality of columns of quadrilateral grids and a plurality of columns of cross units 38. Each column of quadrilateral grids include a plurality of quadrilateral grids arranged substantially in the axial direction, and adjacent quadrilateral grids are connected to each other. Each column of cross units 38 include a plurality of cross units 38 arranged substantially in the axial direction at intervals. The quadrilateral grid is approximately rhombic, or may be in another shape such as a square or a rectangle. Four cross units 38 are correspondingly arranged at four corners of the quadrilateral mesh hole. Each cross unit 38 includes a cross point formed by mutual overlapping of the first directional wave rod 381 and the second directional wave rod 382. In some cross units 38, the first directional wave rod 381 is located outside the second directional wave rod 382. In some cross units 38, the first directional wave rod 381 is located inside the second directional rod 382. In other embodiments, in the first mesh region 32, the first directional wave rods 381 are all located outside the second directional wave rods 382, or the first directional wave rods 381 are all located inside the second directional wave rods 382. In the first mesh region 32, the quadrilateral grids are formed by mutual overlapping or interweaving in a circumferential direction. When the first mesh region 32 and the second mesh region 31 are simultaneously subjected to a radial force, by virtue of a hook structure of the second mesh region 31, the entire second mesh region 31 is not prone to arching and deformation in the radial direction. When the first mesh region 32 is subjected to a large radial force, a middle position first arches to offset the radial force, the second mesh region 31 still maintains the original curved concave shape, and the support units 33 located on two sides of the second mesh region 31 are also compressed and slightly upwarped.

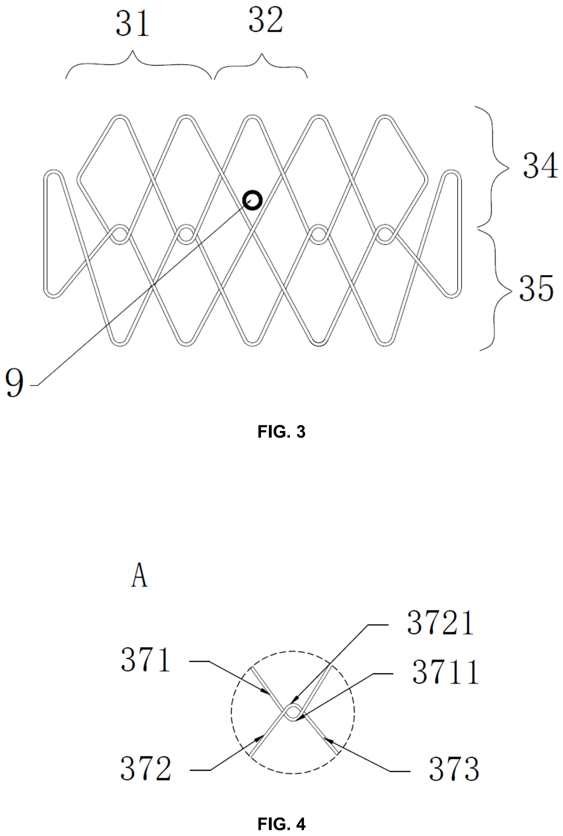

Referring to FIG. 2 and FIG. 4, a first row of grids 34 and a second row of grids 35 in the two second mesh regions 31 are hooked to form one or more hook units 37 arranged at intervals in the radial direction. Each hook unit 37 includes a first hook piece 371 and a second hook piece 372. The first hook piece 371 includes a wave bulging toward a distal end, that is, includes a trough 3711 (also called a distal vertex) and two wave rods 373 connected to the trough 3711. The second hook piece 372 includes a wave bulging toward a proximal end, that is, includes a crest 3721 (also called a proximal vertex) and two wave rods 373 connected to the crest 3721. In this embodiment, the first hook piece 371 and the second hook piece 372 of each hook unit 37 are hooked to each other substantially in the axial direction, so that the trough 3711 of the first hook piece 371 and the crest 3721 of the second hook piece 372 can move relative to each other in the axial direction. It should be noted that "substantially in the axial direction" herein refers to that a connecting line between the distal vertex of the first hook piece 371 and the proximal vertex of the second hook piece 372 is parallel to the axis of the bottom support sheet 3, or the included angle between the connecting line and the axis of the bottom support sheet 3 is less than or equal to 45°.

In other embodiments, a plurality of first mesh regions 32 and a plurality of second mesh regions 31 may be arranged (not shown in the figure). Alternatively, in other embodiments, one first mesh region 32 and one second mesh region 31 are arranged. The quantity of first mesh regions 32 and the quantity of second mesh regions 31 are not limited in the present disclosure.

Referring to FIG. 1, in this embodiment, the bottom support sheet 3 is arranged in the groove 5 at a middle section, and at least an axial end of the bottom support sheet is arranged close to a branch port. It can be seen from the foregoing descriptions that the first row of grids 34 of the bottom support sheet 3 are arranged close to the branch port. It may be understood that the axial end close to the branch port of the bottom support sheet 3, namely, the first row of grids 34, abuts against an edge of the branch port. It may be understood that crests of the first row of grids 34 of the bottom support sheet 3 are expected to abut against the bottom of the corresponding branch port. The bottom support sheet 3 abuts against the branch port, so that the branch port can limit the crests of the bottom support sheet 3, to prevent the crests from being upwarped under a force. In a case of a large blank section between the bottom support sheet 3 and the branch port, when subjected to the radial force, the bottom support sheet 3 is compressed, and the crests at two axial ends are upwarped, resulting in the risk of puncturing a surface membrane and obstructing the branch port.

In another embodiment, a distance is reserved between the axial end close to the branch port of the bottom support sheet 3, namely, the first row of grids 34, and the edge of the branch port, which ranges from 1 mm to 3 mm. This distance should not be excessively large to avoid formation of a large blank section. A smaller spacing distance is appropriately arranged, so that the first row of grids 34 can be deformed within a certain distance toward the branch port without puncturing the membrane.

### Embodiment VI

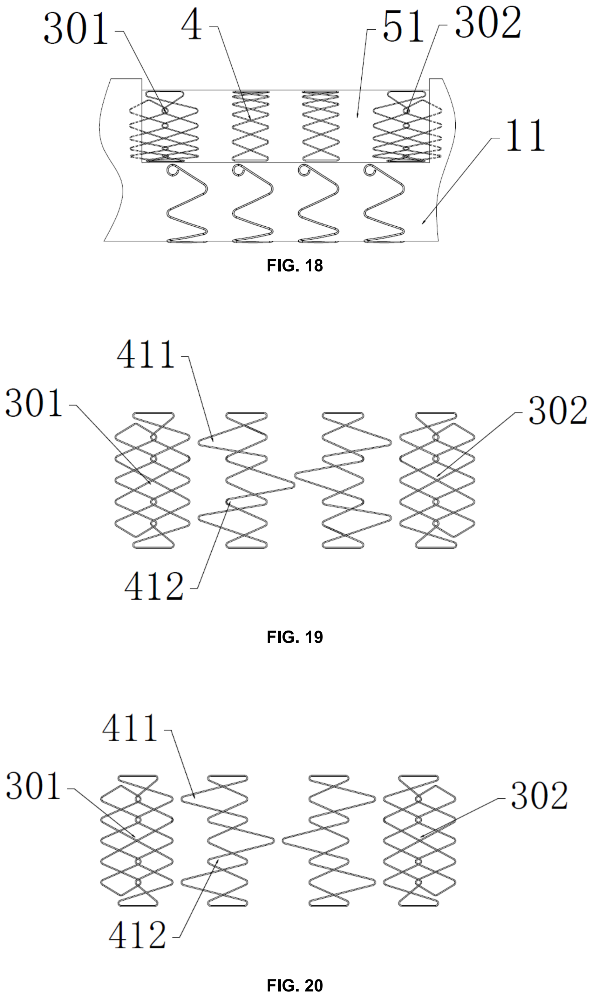

Referring to FIG. 1 and FIG. 18, in this embodiment, the structure of a bottom support sheet 3 is basically the same as those of the bottom support sheets in Embodiment I to Embodiment V. A branch stent 8 includes a stent with two branch ports 81 arranged in an inner cavity of a proximal section 2 and a stent with a single branch port 82 arranged in an inner cavity of a distal section 1. The bottom support sheet 3 includes a proximal bottom support sheet 301 close to the stent with two branch ports 81 and a distal bottom support sheet 302 close to the stent with a single branch port 82. It may be understood that because the stents with branch ports at two ends have different structural characteristics, the structures of the proximal bottom support sheet 301 and the distal bottom support sheet 302 may be identical or different. For example, the quantity of first grids 341 in the first row of grids 34 of the proximal bottom support sheet 301 is equal to the quantity of second grids 351 in the second row of grids 35; and the quantity of first grids 341 in the first row of grids 34 of the distal bottom support sheet 302 is different from the quantity of second grids 351 in the second row of grids 35. Specifically, the quantity of first grids 341 in the first row of grids 34 is less than the quantity of second grids 351 in the second row of grids 35.

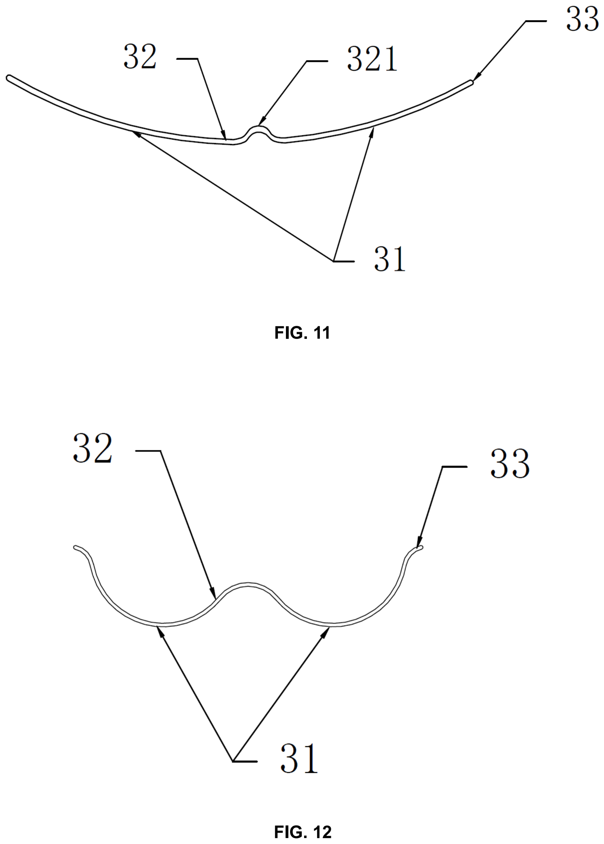

Referring to FIG. 12, FIG. 13, and FIG. 14, the stent with two branch ports 81 includes a first branch stent 811 and a second branch stent 812 that are arranged side by side in a radial direction. A first branch port 8111 is formed at an end close to a groove 5 of the first branch stent 811, and a second branch port 8121 is formed at an end close to the groove 5 of the second branch stent 812. Edges on the sides away from an inner surface of a main stent 10 of the first branch port 8111 and the second branch port 8121 form protruding gaps 813 bulging toward the inner surface of the main stent 10. The protruding gaps 813 and the bottoms of the two branch ports form a slightly curved "W" shape. Through this shape, when the proximal bottom support sheet 301 is deformed, because the proximal bottom support sheet 301 is arranged close to this position, the slightly curved "W" shape can guide deformation of the proximal bottom support sheet 301. The proximal bottom support sheet 301 is expected to form a "W"-like deformation structure, so that the proximal bottom support sheet is more adaptive to the structure of the bottoms of the two branch ports. In this way, the proximal bottom support sheet 301 can provide a sufficient radial support force, and meanwhile is not deformed uncontrollably to obstruct the branch port but deformed into a desired shape under the radial force.

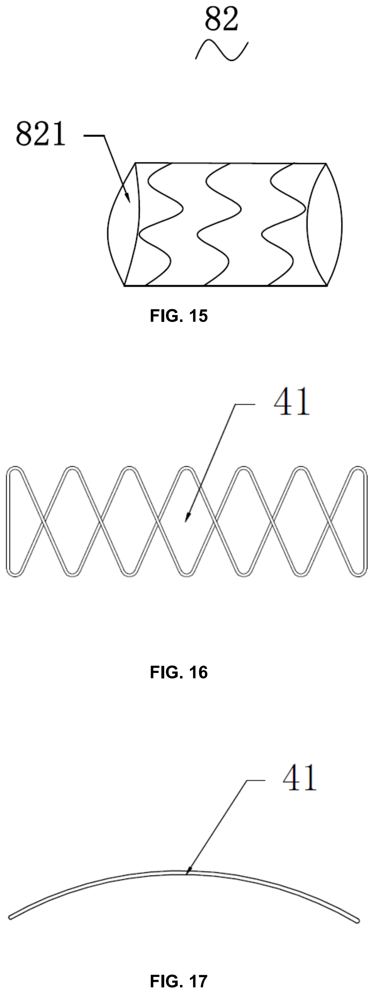

Referring to FIG. 15, it may be understood that a third branch port 821 is formed at an end close to the groove 5 of the stent with a single branch port 82. Because the distal bottom support sheet 302 is arranged close to this position, a single branch port shape of the third branch port 821 can guide deformation of the distal bottom support sheet 302. The distal bottom support sheet 302 is expected to maintain an original arc-shaped structure sinking toward an inner cavity of the main stent 10, so that the distal bottom support sheet does not obstruct the third branch port 821. Further, in a case that the distal bottom support sheet 302 is deformed under compression, through guiding of the single branch port shape of the third branch port 821, the arc-shaped structure, sinking toward the inner cavity of the main stent 10, of the distal bottom support sheet 302 forms a larger curvature, and does not arch in a middle region to form a "W"-like deformation structure. The distal bottom support sheet is not expected to form the "W"-like deformation structure.

In an embodiment, a first mesh region 32 includes at least one column of cross units 38 formed by a plurality of cross units 38, and a connecting line of cross points of the row of cross units 38 coincides with the central axis of the bottom support sheet 3. One column of cross points can ensure that a radial support force at a middle position of at least the first mesh region 32 of the bottom support sheet 3 is weak. At this point, hook structures of second mesh regions 31 on two sides have a good radial support force. The first mesh region 32, by contrast, arches upward and is deformed under radical compression, and the second mesh regions 31 on two sides maintain the original concave shapes due to poor radial deformation capabilities, thereby forming a "W"-like deformation structure.

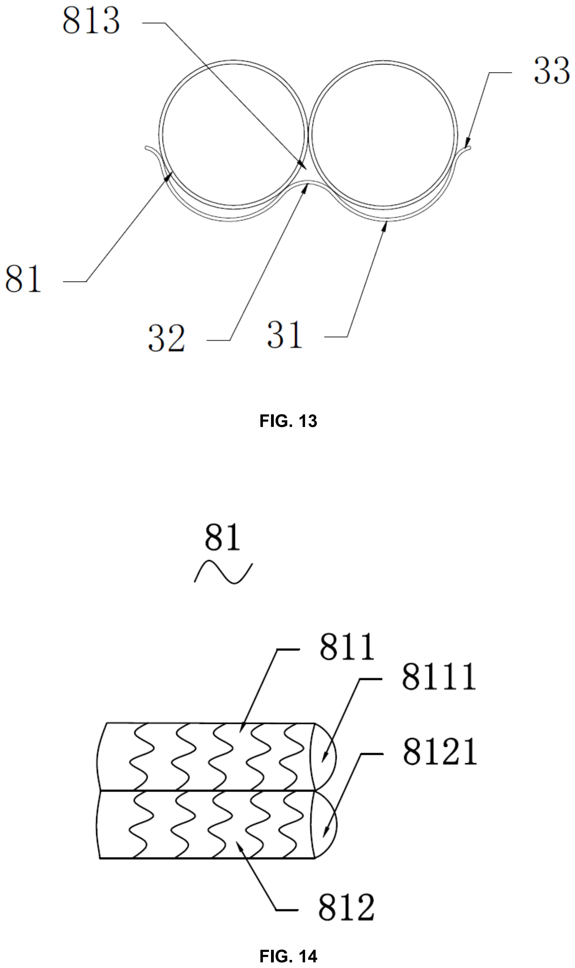

Referring to FIG. 13, it may be understood that if the proximal bottom support sheet 301 is close to or abuts against the first branch port 8111 and the second branch port 8121 of the stent with two branch ports 81, at least part or all of the first mesh region 32 is arranged opposite to the protruding gaps 813 in the axial direction, which ensures that the part of the proximal bottom support sheet 301 that has a good arching deformation capability is guided by the protruding gaps 813, to form a "W"-like deformation structure. However, the two second mesh regions 31 of the proximal bottom support sheet 301 are less likely to undergo arching deformation under the radial force, and maintain the original concave curved structures. This feature meets the requirement that the first branch port 8111 and the second branch port 8121 of the branch stent 8 should remain unobstructed. Further preferably, the second mesh regions 31 of the proximal bottom support sheet 301 are arranged opposite to the first branch stent 811 and the second branch stent 812 in the axial direction, and can conform to the shapes of the branch ports, thereby preventing the proximal bottom support sheet 301 from obstructing the two branch ports under compression.

### Embodiment VII

Referring to FIG. 11 and FIG. 12, in this embodiment, the structure of the bottom support sheet 3 is basically the same as those of the bottom support sheets in Embodiment VI and Embodiment V. A proximal bottom support sheet 301 is bent from the central axis toward two side edges in a circumferential direction to form an arc-shaped structure, and meanwhile, at least a middle portion of a first mesh region 32 forms a protruding portion 321 on an inner concave surface of the arc-shaped structure. Through such an arrangement, firstly, the arc-shaped structure on a main stent 10 is bent toward an inner cavity of the main stent 10 to effectively fit a curved structure of the lower bottom of a branch stent 8, thereby effectively preventing the bottom support sheet 3 from obstructing a branch port. Further, by arranging the protruding portion 321 on an inner curved surface of the arc-shaped structure of the proximal bottom support sheet 301, that is, on a small curvature of the curved structure, if the proximal bottom support sheet 301 forms a W-like structure, the proximal bottom support sheet 301 can better conform to a shape of the two branch ports. It may be understood that the protruding portion 321 may be formed by a part or all of the first mesh region 32 slightly protruding toward the inner concave surface of the small curvature of the arc-shaped structure. The protruding portion 321 may be pre-bent during processing of the proximal bottom support sheet 301, so that the proximal bottom support sheet 3 has a structural feature of a small protrusion. In this way, when the bottom support sheet 3 is installed in a grooved stent 100 and is subjected to a radial force, due to the pre-bent protruding portion 321 of the first mesh region 32, the bottom support sheet can form a W-like deformation structure more easily, to reduce the risk of forming another shape.

In another embodiment, one of the reasons why the first mesh region 32 is more likely to arch compared with second mesh regions 31 on two sides is that the first mesh region 32 is deformed more easily compared with the second mesh regions 31 on two sides under the radial force (not shown in the figure). Therefore, preferably, wire diameters of a first directional wave rod 381 and a second directional wave rod 382 in the first mesh region 32 are less than the wire diameter of the wave rod of the grid in the second mesh region 31. It may be understood that when wave rod pieces are deformed under compression, the force required by bending deformation of a wave rod piece with a smaller wire diameter is smaller than that required by bending deformation of a wave rod piece with a larger wire diameter. Moreover, under the same force, the degree of bending transformation of the wave rod piece with a smaller wire diameter is higher than that of bending deformation of the wave rod piece with a larger wire diameter.

Referring to FIG. 2, in another embodiment, each of first grids 341 and second grids 351 in a first row of grids 34 and a second row of grids 35 in the first mesh region 32 and the second mesh region 31 includes two opposite wave angles (a crest and a trough) in an axial direction, which are connected through wave rods extending in different directions. Preferably, wave widths of the wave angles of the first grids 341 and the second grids 351 in the first mesh region 32 are less than wave widths of the wave angles of the first grids 341 and the second grids 351 in the second mesh regions 31 on two sides. It may be understood that a smaller wave angle indicates a smaller grid enclosed by the wave angles, so that grids formed by mutual overlapping in the first mesh region 32 are smaller than grids in the second mesh regions 31 on two sides. In this way, the first mesh region 32 is more prone to deformation under radical compression, an arching structure is formed more easily at this position, and the bottom support sheet 3 can better form a "W"-like deformation structure.

### Embodiment VIII

Referring to FIG. 1 and FIG. 18, in this embodiment, the structures of the grooved stent 100 and the bottom support sheet 3 are basically the same as those of the grooved stents and the bottom support sheets in Embodiment I to Embodiment VII. A notch is formed at a position corresponding to a groove 5 of a main membrane 11, and a bottom membrane 51 is arranged at the bottom of the groove 5. An axial edge of the bottom membrane 51 is connected to edges of branch ports of two branch stents 8 at a proximal end and a distal end of the groove 5 in an axial direction, and a radial edge of the bottom membrane is connected to the main membrane 11. The bottom membrane 51 is configured to isolate an inner cavity of the grooved stent 100, and is connected to the branch stents 8 at two axial ends to form an accommodation cavity. Preferably, the bottom membrane 51 covers an outer surface away from the inner cavity of the main stent 10 of the bottom support sheet 3. That is, the bottom support sheet 3 is arranged on an inner surface located in the inner cavity of the main stent 10 of the bottom membrane 51, and is connected to the bottom membrane 51 by suturing or bonding. It may be understood that such an arrangement prevents the bottom support sheet 3 from being exposed in the accommodation cavity, thereby preventing the bottom support sheet 3 from being hooked to and pulled by a guide wire entering the accommodation cavity.

Referring to FIG. 3, the grooved stent 100 is further provided with an imaging unit 9. The imaging unit 9 is located in an axial region where a first bottom support sheet 301 is located, is connected to the bottom membrane 51 by suturing or bonding in this region, and is arranged opposite to a protruding gap 813 in the axial direction. Further, when the first bottom support sheet is installed in the groove 5, the imaging unit 9 is opposite to a proximal edge of the groove 5. The imaging unit 9 is configured to indicate the position of the proximal edge of the groove 5 and the position of a stent with two branch ports when the grooved stent is implanted to the human body.

### Embodiment IX

Referring to FIG. 17 and FIG. 18, in this embodiment, the structures of the grooved stent 100 and the bottom support sheet 3 are basically the same as those of the grooved stents and the bottom support sheets in Embodiment V to Embodiment VIII. In this embodiment, the grooved stent 100 further includes a middle support sheet 4, which is arranged in a groove 5 and between a proximal bottom support sheet 301 and a distal bottom support sheet 302. In the middle support sheet 4, at least two waveform units 36 are overlapped with each other to form at least one row of grid support sections 41 extending in a circumferential direction. In a natural unfolded state, the middle support sheet 4 is bent from the central axis toward two side edges in the circumferential direction to form an arc-shaped structure. The middle support sheet 4 arranged in this way can provide sufficient radial support force for a main stent 10. Moreover, the middle support sheet 4 is connected to an inner surface located in an inner cavity of the main stent 10 of a bottom membrane 51 by suturing or bonding, and provides a support structure for the bottom membrane 51 on a surface of the groove 5, which can ensure that the bottom membrane bulges to form sufficient main channel space, but does not bulge excessively, thereby further avoiding irregular deformation.

It may be understood that the middle support 4 may not be arranged in the groove 5 (not shown in the figure). Through such an arrangement, a region between the proximal bottom support sheet 301 and the distal bottom support sheet 302 of the groove 5 is only covered with the bottom membrane 51. The radial support force in this region is small, and irregular deformation is prone to occur when the stent is twisted. When a branch guide wire is introduced, pressure in the groove 5 and the main channel is uniform, so that the membrane in this region does not bulge. After a bridging stent is implanted, because pressure in the main channel is greater than pressure in the groove 5, the membrane in this region bulges to increase the main channel space.

Referring to FIG. 16, in an embodiment, the grid support sections include a plurality of third grids in a radial direction. The wave widths of the third grids in a region between two radial sides of the grid support sections are equal to the wave widths of the third grids on two radial sides.

In another embodiment, the wave widths of the third grids in the region between two radial sides of the grid support sections are less than the wave widths of the third grids on two radial sides. It may be understood that the wave width of the third grid closer to a middle position is smaller, so that the middle position of the middle support sheet 4 has a higher deformation capability. In this way, the middle support sheet 4 can easily form an arch structure under a radial force, which further ensures that the membrane bulges to form larger main channel space.

Referring to FIG. 19 to FIG. 21, in a preferred embodiment, each waveform unit 36 of the grid support sections includes a high wave 411 and a low wave 412. The axial length of the high wave 411 is greater than or equal to the axial length of the low wave 412. The high wave 411 is arranged opposite to and/or staggered with an edge wave angle of an adjacent support sheet. The high waves 411 with a larger axial length can extend to be close to wave angles of the bottom support sheets 3 on two sides, and the high waves 411 of the middle support sheets 4 can extend to be close to each other, so that a blank section between the bottom support sheet 3 and the middle support sheet 4 and a blank section between the middle support sheets 4 can be reduced by the high waves 411. In this way, shrinkage of the groove 5 in an axial direction is effectively avoided.

Referring to FIG. 20, in an embodiment, the high wave 411 of the middle support sheet 4 is opposite to the edge wave angle of the adjacent bottom support sheet 3; and the high wave 411 of the middle support sheet 4 is opposite to the high wave 411 of the adjacent middle support sheet 4.

Referring to FIG. 19, in another embodiment, the high wave 411 of the middle support sheet 4 is staggered with the edge wave angle of the adjacent bottom support sheet 3; and the high wave 411 of the middle support sheet 4 is staggered with the high wave 411 of the adjacent middle support sheet 4.

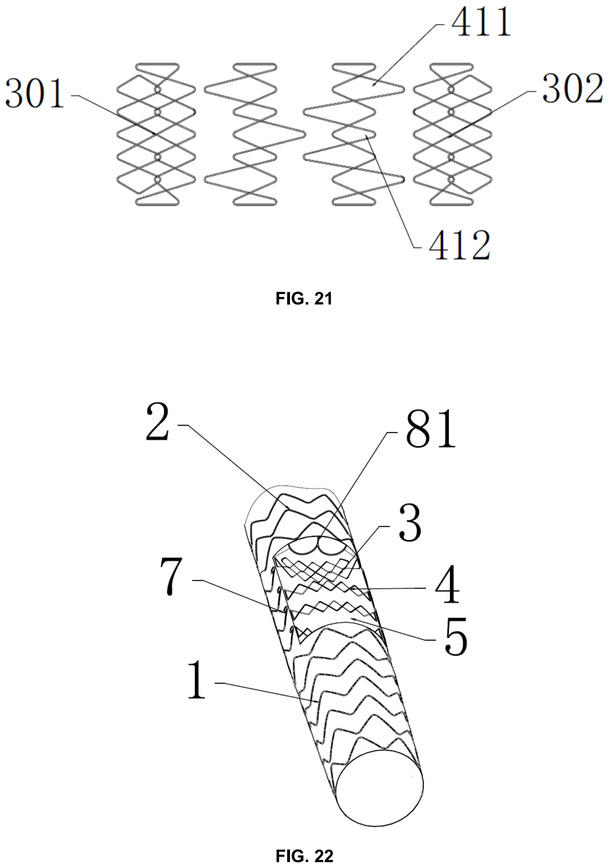

Referring to FIG. 21, in a preferred embodiment, the high wave 411 of the middle support sheet 4 is opposite to the edge wave angle of the adjacent bottom support sheet 3; and the high wave 411 of the middle support sheet 4 is staggered with the high wave 411 of the adjacent middle support sheet 4.

Referring to FIG. 18, in an embodiment, at least two middle support sheets 4 are symmetrically arranged along the central axis of the groove 5.

In a preferred embodiment, the wire size of a weaving wire of the middle support sheet 4 is greater than or equal to the wire size of the bottom support sheet 3, and preferably, is greater than the wire size of the bottom support sheet 3. Through such arrangement, the middle support sheet 4 can provide a larger radial support force to maintain the overall shape of the groove 5.

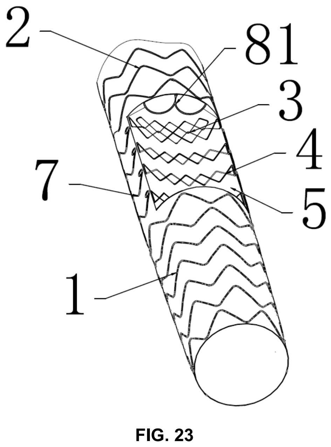

Referring to FIG. 23, in an embodiment, the middle support sheet 4 is bent from the central axis to two side edges in the circumferential direction to form an arc-shaped structure. The bending direction of the arc-shaped structure of the middle support sheet 4 in the groove 5 and the bending direction of an arc-shaped structure of the bottom support sheet 3 are identical, and the middle support sheet and the bottom support sheet are bent toward an inner cavity of the grooved stent 100. By virtue of the same bending direction, the bottom support sheet 3 and the middle support sheet 4 together provide a larger radial support force.

Referring to FIG. 17 and FIG. 22, preferably, when installed in the groove 5, the middle support sheet 4 protrudes relative to an opening of the groove 5 in a direction away from the inner cavity of the main stent 10 to form an arch structure. When installed in the groove 5, the bottom support sheet 3 sinks toward an inner cavity of the main channel to form a concave structure. The arch structure and the concave structure form a stepped structure, which can block blood flow to a certain extent, so that a thrombus is easily formed, and the thrombosis process of the groove 5 is promoted.

In an embodiment, the bottom support sheet 3 is prepared by integrated weaving of shape memory metal wires, or prepared by integrated cutting of a shape memory metal material.

Referring to FIG. 1, FIG. 2, FIG. 3, FIG. 7 to FIG. 9, and FIG. 18 to FIG. 23, in Embodiment I to Embodiment IX, the radial size of the end close to the axial edge of the groove 5 of the bottom support sheet 3 is less than the maximum radial size of the bottom support sheet 3. The end close to the axial edge of the groove 5 of the bottom support sheet 3 is the end close to a branch port of a branch stent 8. Through such an arrangement, at least the end close to branch port of the branch stent 8 of the bottom support sheet 3 can form a structure retracted toward the central axis, namely, a trapezoid-like structure. Because the radial width of the branch stent 8 is less than the radial width of the axial edge of the groove 5, a transition section of a trapezoid-like structure is formed between the branch stent 8 and the axial edge of the groove 5. Through such an arrangement, when the bottom support sheet 3 is installed at the bottom of the groove 5, this structure is similar to the structure of the transition section between the groove 5 and the branch port, so that the bottom support sheet 3 has high adaptability.

In an embodiment, the bottom support sheet 3 includes a mesh region, which includes a first row of grids 34 and a second row of grids 35 that are adjacent to each other in the axial direction. The first row of grids 34 are closer to the axial edge of groove 5 compared with the second row of grids 35. If the radial size of the first row of grids 34 is less than the radial size of the second row of grids 35, the entire bottom support sheet 3 can form a trapezoid-like structure with two axial ends having different sizes, so that the entire bottom support sheet 3 is a structure for directing toward the branch port. This structure is similar to the structure of the transition section between the groove 5 and the branch port, so that the bottom support sheet 3 has high adaptability. When the radial size of the first row of grids 34 is equal to the radial size of the second row of grids 35, the end close to the branch port of the first row of grids 34 is of a wave angle structure, which can ensure that at least the end close to the branch port of the branch stent 8 of the bottom support sheet 3 forms a structure retracted toward the central axis.

The radial size of the first row of grids 34 is less than the radial size of the axial edge of the groove 5, because the radial width of the branch stent 8 is less than the radial width of the axial edge of the groove 5, the radial side of the bottom support sheet 3 needs to be connected to a radial side of the groove 5, and the radial size of the bottom support sheet at least needs to be equal to the radial size of the groove 5. The radial size of the first row of grids 34 is less than the radial size of the axial edge of the groove 5, which also ensures that the first row of grids 34 and the second row of grids 35 of the bottom support sheet 3 form a structure adaptive to the trapezoidal transition section, thereby ensuring adaptability of the installed bottom support sheet 3, and avoiding the problem of puncturing the membrane and irregular deformation affecting the shape of the branch port due to low adaptability of the bottom support sheet 3.

The foregoing specific embodiments are merely some embodiments of the present disclosure, and are not intended to limit the present disclosure. The description cannot be an exhaustive list of all embodiments of the present disclosure, and some features of the foregoing different embodiments may be replaced or combined with each other. Those skilled in the art may also make simple substitutions according to actual needs. The concept of the present disclosure is subject to the claimed scope of protection.

## Claims

1. A grooved stent, **characterized in that** the grooved stent is of a hollow tubular structure with ports at two ends and comprises a main stent and a bottom support sheet, a groove is formed in a surface of the main stent, and the bottom support sheet is arranged at the bottom of the groove and close to an axial edge of the groove; and the radial size of an end close to the axial edge of the groove of the bottom support sheet is less than the maximum radial size of the bottom support sheet.

2. The grooved stent according to claim 1, **characterized in that** the bottom support sheet comprises a mesh region, which comprises a first row of grids and a second row of grids that are adjacent to each other in an axial direction; and the first row of grids are closer to the axial edge of the groove compared with the second row of grids, and the radial size of the first row of grids is less than or equal to the radial size of the second row of grids, and is less than the radial size of the axial edge of the groove.

3. The grooved stent according to claim 1 or 2, **characterized in that** the bottom support sheet comprises a first mesh region and two second mesh regions respectively connected to two sides of the first mesh region in a radial direction; the first mesh region and the second mesh regions comprise the first row of grids and the second row of grids that are adjacent to each other in the axial direction; and the first row of grids and the second row of grids are hooked in the two second mesh regions; and the first row of grids and the second row of grids are connected in the first mesh region to form cross units.

4. The grooved stent according to claim 3, **characterized in that** the main stent comprises a proximal section, a distal section, and a middle section between the proximal section and the distal section, and the groove is formed in the middle section; and a branch stent is arranged in an inner cavity of the proximal section and/or the distal section and close to the groove, and comprises a branch port, the bottom support sheet is arranged in the groove, and at least an axial end of the bottom support sheet is arranged close to the branch port.

5. The grooved stent according to claim 4, **characterized in that** the branch stent comprises a stent with two branch ports; and the bottom support sheet close to the stent with two branch ports is a first bottom support sheet, the stent with two branch ports comprises a first branch stent and a second branch stent that are arranged side by side in the radial direction, a first branch port is formed at an end close to the groove of the first branch stent, a second branch port is formed at an end close to the groove of the second branch stent, wherein edges on sides that are away from an inner surface of the main stent of the first branch port and the second branch port form protruding gaps bulging toward the inner surface of the main stent, and the first mesh region of the first bottom support sheet is arranged opposite to the protruding gaps in the axial direction.

6. The grooved stent according to claim 1 or 2, **characterized in that** the bottom support sheet comprises the mesh region; and a radial side of the mesh region is connected to at least one deformable support unit, the support unit comprises at least one movable end portion, a gap is reserved between the movable end portion and the mesh region, and the movable end portion is capable of moving relative to the mesh region in at least a radial direction.

7. The grooved stent according to claim 6, **characterized in that** the support unit comprises a fixed end portion, a connecting rod, and a buffer rod connected to the connecting rod, and is connected to the mesh region through the fixed end portion, one end of the connecting rod is connected to the mesh region to form the fixed end portion, the other end of the connecting rod extends away from the mesh region and toward a radial edge of the groove, to form the gap with the mesh region, and the buffer rod is capable of moving relative to the connecting rod.

8. The grooved stent according to claim 6, **characterized in that** the support unit comprises a fixed end portion, at least two connecting rods, and at least one buffer rod, and is connected to the mesh region through the fixed end portion, wherein the ends connected to the mesh region of two connecting rods are intersected to form the fixed end portion, and the other ends of the two connecting rods extend obliquely from the fixed end portion in directions away from each other and are respectively connected to two ends of the buffer rod.

9. A grooved stent, **characterized in that** the grooved stent is of a hollow tubular structure with ports at two ends and comprises a main stent and a bottom support sheet, a groove is formed in a surface of the main stent, and the bottom support sheet is arranged at the bottom of the groove and close to an axial edge of the groove; the bottom support sheet comprises a mesh region; and a radial side of the mesh region is connected to at least one deformable support unit, the support unit comprises at least one movable end portion, a gap is reserved between the movable end portion and the mesh region, and the movable end portion is capable of moving relative to the mesh region at least in a radial direction.

10. The grooved stent according to claim 9, **characterized in that** the support unit comprises a fixed end portion, and is connected to the mesh region through the fixed end portion.

11. The grooved stent according to claim 10, **characterized in that** the support unit comprises a connecting rod, one end of the connecting rod is connected to the mesh region to form the fixed end portion, and the other end of the connecting rod extends away from the mesh region and toward a radial edge of the groove, to form the gap with the mesh region.

12. The grooved stent according to claim 11, **characterized in that** the support unit further comprises a buffer rod connected to the connecting rod, and the buffer rod is capable of moving relative to the connecting rod.

13. The grooved stent according to claim 10, **characterized in that** the support unit comprises at least two connecting rods and at least one buffer rod, wherein the ends connected to the mesh region of two connecting rods are intersected to form the fixed end portion, and the other ends of the two connecting rods extend obliquely from the fixed end portion in directions away from each other and are respectively connected to two ends of the buffer rod.

14. The grooved stent according to claim 10, **characterized in that** the support unit comprises at least two connecting rods and a plurality of buffer rods, and the plurality of buffer rods are connected end to end to form a polygonal rod; and the ends connected to the mesh region of two connecting rods are intersected to form the fixed end portion, and the other ends of the two connecting rods extend obliquely from the fixed end portion in directions away from each other and are respectively connected to head and tail ends of the polygonal rod to form a polygonal grid.

15. The grooved stent according to claim 13 or 14, **characterized in that** the included angle between one connecting rod and the buffer rod is greater than or equal to the included angle between the other connecting rod and the buffer rod.

16. The grooved stent according to claim 13 or 14, **characterized in that** the lengths of the two connecting rods are equal or the length of a connecting rod is greater than the length of the other connecting rod.

17. The grooved stent according to claim 13 or 14, **characterized in that** the minimum distance between the end away from the mesh region of one connecting rod and the mesh region is greater than the minimum distance between the end away from the mesh region of the other connecting rod and the mesh region.

18. The grooved stent according to claim 9, **characterized in that** the mesh region comprises a first row of grids and a second row of grids that are adjacent to each other in an axial direction; and the first row of grids are closer to the axial edge of the groove compared with the second row of grids, and the support unit is connected to a radial side of the second row of grids.

19. The grooved stent according to claim 18, **characterized in that** the first row of grids comprise a plurality of first grids arranged in the radial direction; the second row of grids comprise a plurality of second grids arranged in the radial direction; and one first grid corresponds to one second grid in the axial direction, and the quantity of first grids is less than or equal to the quantity of second grids.

20. The grooved stent according to claim 19, **characterized in that** the first row of
grids comprise side grids on two radial sides and a middle grid between the two side grids, and the distance between the middle grid and the axial edge of the groove is less
than the distance between each side grid and the axial edge of the groove.
